# EUROPEAN PATENT SPECIFICATION

(11) **EP 3 215 229 B1**
(45) Date of publication and mention of the grant of the patent: **01.04.2020**
(21) Application number: 15820779.5
(22) Date of filing: 17.12.2015
(51) Int. Cl.: A61Q 11/00, A61K 8/19, A61K 8/362, A61K 31/191, A61K 33/00, A61K 31/194, A61K 9/00, A61P 43/00

(54) **MOUTHRINSE FORMULATIONS**
MUNDSPÜLUNGSFORMULIERUNGEN
FORMULATIONS D'EAU DENTAIRE

(30) Priority: 18.12.2014 US 201462093545 P
(43) Date of publication of application: 13.09.2017
(73) Proprietor: GABA International Holding GmbH, 4106 Therwil (CH)
(72) Inventor: HINRICHS, Ruth, CH-4106 Therwil (CH); MATUR, Turan, CH-4102 Binningen (CH); BRUNELLA, André, 4143 Dornach (CH); HESS, Sylvia, CH-4434 Hölstein (CH)
(74) Representative: Wibbelmann, Jobst
(86) International application number: PCT/US2015/066304
(87) International publication number: WO 2016/100628

(56) References cited:
- US-A- 3 282 792
- US-A- 4 267 164
- US-A- 4 335 102
- US-A- 4 363 794

## Description

The present invention relates to oral care compositions comprising ionic tin and in particular to stannous-containing mouth rinse formulations.

### BACKGROUND

Tin ions such as stannous ions have been incorporated into oral care compositions including dentifrices and mouth rinses in order to provide anti-caries benefits and in order to treat and/or prevent gingivitis. However, mouth rinse formulations comprising tin ions can be unstable and can have a tendency to form a white precipitate upon storage. This is the result of the formation of the highly insoluble salt stannic oxide, which precipitates from the formulation.

It would be desirable to be able to formulate stable oral care mouth rinse compositions comprising tin ions.

US 3,282,792 A discloses an aqueous composition to which has been added between about 0.1% and 5% of stannous fluoride and between about 0.05% and 10% of a stabilizer therefore selected from the class consisting of malic acid and the water-soluble salts of citric acid and hydroxyl substituted aliphatic dicarboxylic acids.

US 4,363,794 A discloses an oral composition for caries prophylaxis comprising a stannous salt, a water-soluble fluoride salt selected from the group consisting of sodium fluoride, potassium fluoride and mixtures thereof, and an orally acceptable acid selected from the group consisting of a monocarboxylic acid, a dicarboxylic acid, a tricarboxylic acid, a mineral acid and mixtures thereof, wherein said stannous salt and said orally acceptable acid are present in specific amounts.

US 4,335,102 A discloses an oral composition for caries prophylaxis, comprising: a specific highly soluble stannous compound, said highly soluble stannous compound being present in the range of 0.03% to 5% by weight of the composition as stannous tin; a specific difficulty soluble stannous compound, said difficulty soluble stannous compound being present in the range of 0.05% to 10% by weight of the composition as stannous tin; and 6.1 % to 20% by weight of the composition of a specific phytic acid compound, said composition being acidic.

US 4,267,164 A discloses an effervescent stannous fluoride containing dental tablet comprising stannous fluoride and an effervescent couple consisting essentially of malic acid and an alkali metal bicarbonate in a weight ratio of about 3:2.

### BRIEF SUMMARY

It has now surprisingly been discovered that the combination of tin ions with 0.05 to 0.30 weight % of a specific aliphatic di- or tri-carboxylic acid in a mouth rinse provides a highly stable oral care mouth rinse formulation that combines oral care benefits with storage stability.

According to a first aspect of the present invention there is provided an oral care mouth rinse composition comprising: (i) at least one source of ionic tin; (ii) at least one aliphatic di- or tri-carboxylic acid in free or salt form wherein the at least one aliphatic di- or tri-carboxylic acid in free or salt form is present in an amount of from 0.05 to 0.30 weight % based on the total weight of the oral care composition, wherein the at least one aliphatic di- or tri-carboxylic acid comprises malic acid in an amount of 0.12 to 0.25 weight % based on the total weight of the oral care composition; and (iii) amine fluoride.

Preferably, the oral care mouth rinse composition comprises 0.10 to 0.20 weight % of said aliphatic di- or tri-carboxylic acid in free or salt form. Further optionally the oral care mouth rinse composition comprises 0.12 to 0.15 weight % of said aliphatic di- or tri-carboxylic acid in free or salt form.

According to the invention, the at least one aliphatic di- or tri-carboxylic acid comprises malic acid in an amount of 0.12 to 0.25 weight % based on the total weight of the oral care composition. Further optionally the oral care mouth rinse composition comprises 0.12 to 0.15 weight % malic acid.

Optionally the source of ionic tin is selected from the group comprising stannous ion sources, stannic ion sources and combinations thereof. Optionally the source of ionic tin is selected from the group comprising stannous fluoride, stannous chloride, stannic fluoride, stannic chloride, stannic acetate, stannous acetate and combinations thereof. Optionally the oral care mouth rinse composition comprises at least one stannous ion source. Optionally the source of ionic tin is selected from the group comprising stannous fluoride, stannous chloride, stannous acetate and combinations thereof.

Optionally the concentration of ionic tin is from 0.01 to 0.10 weight % based on the total weight of the oral care mouth rinse composition. Further optionally the concentration of ionic tin is from 0.02 to 0.08 weight % based on the total weight of the oral care mouth rinse composition. Further optionally the concentration of ionic tin is from 0.03 to 0.06 weight % based on the total weight of the oral care mouth rinse composition. Further optionally the concentration of ionic tin is 0.04 weight % based on the total weight of the oral care mouth rinse composition.

Optionally the at least one source of ionic tin comprises stannous fluoride.

The oral care mouth rinse composition further comprises a non-stannous fluoride ion source, which is amine fluoride. Further optionally the oral care mouth rinse composition comprises sodium fluoride, potassium fluoride, sodium monofluorophosphate, sodium fluorosilicate, ammonium fluorosilicate, ammonium fluoride and combinations thereof. Further optionally the oral care mouth rinse composition comprises sodium fluoride. Further optionally the oral care mouth rinse composition comprises amine fluoride or sodium fluoride in an amount corresponding to 45 to 1500 ppm fluoride based on the total weight of the composition. Further optionally the oral care mouth rinse composition comprises 0.06 to 0.33 weight % amine fluoride or 0.01 to 0.33 weight % sodium fluoride or a combination thereof, based on the total weight of the composition.

Optionally the oral care mouth rinse composition comprises 5 to 1500 ppm fluoride.

Optionally the oral care mouth rinse composition comprises 0.03 to 0.08 weight % stannous fluoride and 0.10 to 0.25 weight % amine fluoride.

Optionally the oral care mouth rinse composition comprises 0.04 to 0.08 weight % stannous fluoride and 0.10 to 0.25 weight % N,N,N'-tris(2-hydroxyethyl)-N'-octadecyl-1,3-diaminopropane dihydrofluoride.

According to a further aspect of the invention there is provided a method to reduce or inhibit formation of dental caries, reduce, repair or inhibit pre-carious lesions of the enamel, reduce or inhibit demineralization and promote remineralization of the teeth reduce hypersensitivity of the teeth, reduce or inhibit gingivitis, promote healing of sores or cuts in the oral cavity, reduce levels of acid producing bacteria, reduce or inhibit microbial biofilm formation in the oral cavity, reduce or inhibit plaque formation in the oral cavity, promote systemic health and/or clean teeth and oral cavity, comprising applying an effective amount of an oral care mouth rinse composition as described herein to the oral cavity of a subject in need thereof.

According to a further aspect of the present invention there is provided an oral care mouth rinse composition for use in a method to reduce or inhibit formation of dental caries, reduce, repair or inhibit pre-carious lesions of the enamel, reduce or inhibit demineralization and promote remineralization of the teeth, reduce hypersensitivity of the teeth, reduce or inhibit gingivitis, promote healing of sores or cuts in the oral cavity, reduce levels of acid producing bacteria, reduce or inhibit microbial biofilm formation in the oral cavity, reduce or inhibit plaque formation in the oral cavity, promote systemic health clean teeth and oral cavity.

According to a further aspect of the present invention there is provided use of an oral care mouth rinse composition to reduce or inhibit formation of dental caries, reduce, repair or inhibit pre-carious lesions of the enamel, reduce or inhibit demineralization and promote remineralization of the teeth, reduce hypersensitivity of the teeth, reduce or inhibit gingivitis, promote healing of sores or cuts in the oral cavity, reduce levels of acid producing bacteria, reduce or inhibit microbial biofilm formation in the oral cavity, reduce or inhibit plaque formation in the oral cavity, promote systemic health and/or clean teeth and oral cavity in a subject in need thereof.

According to a further aspect of the present invention there is also provided a method of stabilizing ionic tin in an oral care mouth rinse composition comprising at least one source of ionic tin, wherein the method comprises formulating the oral care mouth rinse composition to comprise at least one aliphatic di- or tri-carboxylic acid in free or salt form in an amount of from 0.05 to 0.30 weight % based on the total weight of the oral care composition. Optionally the concentration of ionic tin in the oral care mouth rinse composition is from 0.01 to 0.10 weight % based on the total weight of the oral care mouth rinse composition. Optionally the composition further comprises 0.10 to 0.25 weight % amine fluoride.

According to a further aspect of the present invention there is also provided a method of reducing staining of surfaces in the oral cavity resulting from use of an oral care mouth rinse comprising at least one source of ionic tin, wherein the method comprises formulating the oral care mouth rinse composition to comprise at least one aliphatic di- or tri-carboxylic acid in free or salt form in an amount of from 0.05 to 0.30 weight % based on the total weight of the oral care composition. Optionally the concentration of ionic tin in the oral care mouth rinse composition is from 0.01 to 0.10 weight % based on the total weight of the oral care mouth rinse composition. Optionally the composition further comprises 0.10 to 0.25 weight % amine fluoride.

### DETAILED DESCRIPTION

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material.

It has surprisingly been found that aliphatic di- or tri-carboxylic acids can be used in tin-containing mouth rinse formulations to improve the stability of the ionic tin. The inventors of the present application have discovered that such acids can reduce or prevent the formation of insoluble precipitate and/or a turbid appearance upon storage and ageing. Previously, mouth rinse formulations comprising ionic tin have had a tendency to form sediments and/or develop a turbid appearance when stored, for example at 25 °C for three or more months.

Surprisingly, formulations comprising aliphatic di- or tri-carboxylic acids also showed a reduction in staining. Previously, compositions comprising ionic tin have shown a tendency to stain teeth *in vivo.*

Mouth rinse (or mouthwash) compositions comprise one or more oral care active component in a liquid carrier. Typically, mouth rinse compositions are formulated using a solvent such as water. They may be used to deliver oral care actives to the oral cavity of a consumer and may be used either before or after brushing and/or flossing.

The mouth rinse compositions of the present invention comprise a source of ionic tin. In certain embodiments the source of ionic tin comprises a stannous or stannic salt, or a combination thereof. In certain embodiments the source of ionic tin comprises a stannous salt. In certain embodiments the source of ionic tin is selected from water-soluble stannous salts such as stannous fluoride, stannous chloride, stannous acetate and combinations thereof. In certain embodiments the source of ionic tin comprises stannous fluoride, stannous chloride or combinations thereof. In certain preferred embodiments, the source of ionic tin is stannous fluoride. In certain embodiments separate soluble stannous and fluoride salts may be used to provide stannous fluoride in situ. Alternatively, stannous fluoride salt may be added to the composition directly.

In one embodiment, the present invention provides an oral mouth rinse composition (Composition 1) comprising: (i) at least one source of ionic tin; (ii) at least one aliphatic di- or tri-carboxylic acid in free or salt form wherein the at least one aliphatic di- or tri-carboxylic acid in free or salt form is present in an amount of from 0.05 to 0.30 weight % based on the total weight of the oral care composition, wherein the at least one aliphatic di- or tri-carboxylic acid comprises malic acid in an amount of 0.12 to 0.25 weight % based on the total weight of the oral care composition; and (iii) amine fluoride.. In preferred embodiments, the present invention provides Compositions as follows:
1.1 Composition 1, comprising 0.10 to 0.25 weight % aliphatic di- or tri-carboxylic acid in free or salt form.
1.2 Composition 1 or 1.1, comprising 0.10 to 0.20 weight % aliphatic di- or tri-carboxylic acid in free or salt form.
1.3 Composition 1, 1.1 or 1.2, comprising 0.12 to 0.15 weight % aliphatic di- or tri-carboxylic acid in free or salt form.
1.4 Composition 1 or 1.1 *et seq.* wherein the source of ionic tin is selected from the group comprising stannous ion sources, stannic ion sources and combinations thereof.
1.5 Composition 1 or 1.1 *et seq.* wherein the source of ionic tin is selected from the group comprising stannous fluoride, stannous chloride, stannic fluoride, stannic chloride, stannic acetate, stannous acetate and combinations thereof.
1.6 Composition 1 or 1.1 *et seq.* wherein the composition comprises at least one stannous ion source.
1.7 Composition 1 or 1.1 *et seq.* wherein the source of ionic tin is selected from the group comprising stannous fluoride, stannous chloride, stannous acetate and combinations thereof.
1.8 Composition 1 or 1.1 *et seq.* wherein the concentration of ionic tin is from 0.01 to 0.10 weight % based on the total weight of the oral care mouth rinse composition.
1.9 Composition 1 or 1.1 *et seq.* wherein the concentration of ionic tin is from 0.02 to 0.08 weight % based on the total weight of the oral care mouth rinse composition.
1.10 Composition 1 or 1.1 *et seq.* wherein the concentration of ionic tin is from 0.03 to 0.06 weight % based on the total weight of the oral care mouth rinse composition.
1.11 Composition 1 or 1.1 *et seq.* wherein the concentration of ionic tin is 0.04 weight % based on the total weight of the oral care mouth rinse composition.
1.12 Composition 1 or 1.1 *et seq.* wherein the source of ionic tin comprises stannous fluoride.
1.13 Composition 1 or 1.1 *et seq.* comprising sodium fluoride, potassium fluoride, sodium monofluorophosphate, sodium fluorosilicate, ammonium fluorosilicate, ammonium fluoride and combinations thereof.
1.14 Composition 1 or 1.1 *et seq.* comprising sodium fluoride.
1.15 Composition 1 or 1.1 *et seq.* comprising amine fluoride or sodium fluoride in an amount corresponding to 45 to 1500 ppm fluoride based on the total weight of the composition.
1.16 Composition 1 or 1.1 *et seq.* comprising 0.06 to 0.33 weight % amine fluoride or 0.01 to 0.33 weight % sodium fluoride or a combination thereof based on the total weight of the composition.
1.17 Composition 1 or 1.1 *et seq.* comprising 5 to 1500 ppm fluoride.
1.18 Composition 1 or 1.1 *et seq.* comprising 0.04 to 0.08 weight % stannous fluoride and 0.10 to 0.25 weight % N,N,N'-tris(2-hydroxyethyl)-N'-octadecyl-1,3-diaminopropane dihydrofluoride.
1.19 Composition 1 or 1.1 *et seq.* comprising 0.03 to 0.08 weight % stannous fluoride and 0.10 to 0.25 weight % N,N,N'-tris(2-hydroxyethyl)-N'-octadecyl-1,3-diaminopropane dihydrofluoride.
1.20 Composition 1, wherein the composition comprises an amine fluoride in an amount of from 0.10 to 0.25% by weight of the composition.
1.21 Composition 1 or 1.20, wherein the amine fluoride is selected from N,N,N'-tris(2-hydroxyethyl)-N'-octadecyl-1,3-diaminopropane dihydrofluoride, 9-octadecenylamine hydrofluoride, or a combination thereof.
1.22 Composition 1 or 1.20 wherein the amine fluoride is a mixture of N,N,N'-tris(2-hydroxyethyl)-N'-octadecyl-1,3-diaminopropane dihydrofluoride, and 9-octadecenylamine hydrofluoride.
1.23 Any of Compositions 1 to 1.22 for use in a method to:
   (i) reduce or inhibit formation of dental caries,
   (ii) reduce, repair or inhibit pre-carious lesions of the enamel,
   (iii) reduce or inhibit demineralization and promote remineralization of the teeth,
   (iv) reduce hypersensitivity of the teeth,
   (v) reduce or inhibit gingivitis,
   (vi) promote healing of sores or cuts in the oral cavity,
   (vii) reduce levels of acid producing bacteria,
   (viii) reduce or inhibit microbial biofilm formation in the oral cavity,
   (ix) reduce or inhibit plaque formation in the oral cavity,
   (x) promote systemic health, or
   (xi) clean teeth and oral cavity.

In certain embodiments the concentration of ionic tin in the oral care mouth rinse composition is from 0.01 to 0.10 weight % based on the total weight of the oral care mouth rinse composition. In certain embodiments the concentration of ionic tin in the oral care mouth rinse composition is from 0.02 to 0.08 weight % or from 0.03 to 0.06 weight %. In certain embodiments the concentration of ionic tin is about 0.040 weight % based on the total weight of the oral care mouth rinse composition.

The compositions of the present invention comprise at least one aliphatic di-or tri-carboxylic acid in free or salt form. In certain embodiments the carboxylic acid may be added as the free acid or as a salt. In certain embodiments, the carboxylic acid is added as the sodium or potassium salt. In certain embodiments the carboxylic acid is an α-hydroxy acid. In certain embodiments the carboxylic acid is a α-hydroxy di-carboxylic acid. The carboxylic acid may be a racemic mixture of the L- and D-form of an aliphatic di- or tri-carboxylic acid. In certain embodiments the carboxylic acid is a racemic mixture of L-malic acid and D-malic acid.

The aliphatic di- or tri-carboxylic acid in free or salt form is present in an amount of from 0.05 to 0.30 weight % based on the total weight of the oral care composition. In certain embodiments the composition comprises 0.10 to 0.25 weight %, 0.10 to 0.20 weight %, 0.10 to 0.15 weight %, 0.12 to 0.25 weight %, 0.12 to 0.20, or 0.12 to 0.15 weight % aliphatic di- or tri-carboxylic acid in free or salt form. In certain embodiments the composition comprises 0.12 to 0.20, or 0.12 to 0.15 weight % malic acid.

According to the invention, the mouth rinse composition comprises a non-stannous fluoride ion source i.e. a source of fluoride ions that is not a stannous salt. In certain embodiments the compositions of the invention comprise sodium fluoride, potassium fluoride, sodium monofluorophosphate, sodium fluorosilicate, ammonium fluorosilicate, ammonium fluoride and/or combinations thereof. In certain embodiments the composition comprises a fluoride ion source in a concentration sufficient to supply about 5 ppm to about 1,500 ppm fluoride ions, for example from about 25 ppm to about 1,500 ppm, from about 100 ppm to about 1,500 ppm, from about 200 ppm to about 550 ppm or from about 250 ppm to about 500 ppm.

In certain embodiments, the mouth rinse composition comprises sodium fluoride, and/or a combination thereof. In certain embodiments the composition comprises OLAFLUR (N,N,N'-tris(2-hydroxyethyl)-N'-octadecyl-1,3-diaminopropane dihydrofluoride) or DECTAFLUR (9-octadecenylaminehydrofluoride) in an amount of from 0.10 to 0.25 weight % or a mixture of both OLAFLUR and DECTAFLUR.

According to the invention, the mouth rinse composition comprises a combination of a source of ionic tin and amine fluoride, for example a stannous salt and amine fluoride. In certain embodiments the mouth rinse composition comprises stannous fluoride and amine fluoride. In certain embodiments the mouth rinse composition comprises stannous fluoride and OLAFLUR (N,N,N'-tris(2-hydroxyethyl)-N'-octadecyl-1,3-diaminopropane dihydro fluoride).

In certain embodiments the compositions of the invention may also comprise a salt of pyrrolidone carboxylic acid (also known as PCA, 2-pyrrolidone-5-carboxylic acid; 5-oxoproline; pidolic acid or pyroglutamic acid). In certain embodiments the compositions of the invention may comprise a salt of the L-enantiomer of pyrrolidone carboxylic acid, for example zinc pyrrolidone carboxylate, copper pyrrolidone carboxylate, magnesium pyrrolidone carboxylate or manganese pyrrolidone carboxylate. In certain embodiments the compositions of the invention may comprise zinc L-pyrrolidone carboxylate. The salt of pyrrolidone carboxylic acid may be present in an amount of from 0.01 to 0.75 weight %, for example from 0.05 to 0.50 weight % or from 0.10 to 0.20 weight.

In any of the above embodiments, the compositions may further comprise a flavoring agent. The flavoring agent may comprise one or more essential oils as well as various flavoring aldehydes, esters and/or alcohols. In certain embodiments, the flavoring agent comprises one or more essential oil selected form oils of peppermint, wintergreen, sassafras, clove, sage, eucalyptus, marjoram, cinnamon, lemon, lime, grapefruit and orange. In certain embodiments, the flavoring agent comprises oils of peppermint and spearmint. In certain embodiments the composition comprises from 0.01 to 2.0 weight %, 0.05 to 1.0 weight % or 0.10 to 0.20 weight % flavoring agent based on the total weight of the composition.

In any of the above embodiments, the compositions may further comprise at least one surfactant. Any orally acceptable surfactant such as a nonionic, anionic or amphoteric surfactant may be used. Optionally a surfactant may be present in an amount of from 0.01 weight % to 10 weight %, for example from 0.05 to 5 weight % or from 0.10 to 2.0 weight % based on the total weight of the composition.

In any of the above embodiments, the compositions may further comprise a sweetener such as, for example, sodium saccharin. One or more sweeteners may be present in an amount of from 0.005 weight % to 5 weight % by total weight of the composition, for example, 0.05 weight % to 0.1 weight %.

In any of the above embodiments, the compositions may further comprise at least one colorant. Colorants may include pigments, dyes, lakes and agents imparting a particular luster or reflectivity such as pearling agents. Any orally acceptable colorant can be used. One or more colorants may optionally be present in the compositions in an amount of from 0.0001 weight % to 20 weight %, for example from 0.0001 weight % to 1 weight % or from about 0.0001 weight % to 0.010 weight %.

In any of the above embodiments, the compositions may further comprise a polyhydric alcohol such as glycerin, sorbitol, xylitol, propylene glycol and combinations thereof. In certain embodiments the compositions may optionally comprise form about 0.10 to about 10 weight % polyhydric alcohol based on the total weight of the composition. In certain embodiments the compositions may comprise from 0.50 to 10 weight % xylitol, for example from 0.50 to 7.0 weight % xylitol. In certain embodiments the compositions may comprise from 0.50 to 5.0 weight % glycerin, for example 1.0 to 3.5 weight % glycerin. In certain embodiments the compositions may comprise 0.10 to 1.0 weight % propylene glycol. In any of the above embodiments, the compositions may comprise 0.50 to 7.0 weight % xylitol and 1.0 to 3.5 weight % glycerin.

In any of the above embodiments, the compositions may further comprise one or more polymers such as polyethylene glycols, polyvinylmethyl ether maleic acid copolymers and polysaccharides (e.g. cellulose derivatives such as carboxymethyl cellulose or microcrystalline cellulose, or polysaccharide gums such as xanthan gum or carrageenan gum). Such polymers may be present in any of the compositions of the invention in an amount of from 0.05 to 5.0 weight %. In any of the above embodiments, the compositions may further comprise a pH adjuster. For example the compositions may comprise an acid or base in an amount sufficient to adjust the pH of the compositions such that the compositions have a pH of from 4.0 to 8.0. In certain embodiments, the compositions may include aqueous potassium hydroxide as a pH adjuster. In certain embodiments the compositions may include aqueous potassium hydroxide in an amount such that the pH of the composition is from 5.5 to 7.5. In certain embodiments aqueous potassium hydroxide is added to the compositions to adjust the pH to around 6.5 to 7.5, for example about 7.0.

In another aspect, the present invention provides the oral care mouth rinse composition of the invention for use in a method of

:
(i) reduce or inhibit formation of dental caries,
(ii) reduce, repair or inhibit pre-carious lesions of the enamel,
(iii) reduce or inhibit demineralization and promote remineralization of the teeth,
(iv) reduce hypersensitivity of the teeth,
(v) reduce or inhibit gingivitis,
(vi) promote healing of sores or cuts in the oral cavity,
(vii) reduce levels of acid producing bacteria,
(viii) reduce or inhibit microbial biofilm formation in the oral cavity,
(ix) reduce or inhibit plaque formation in the oral cavity,
(x) promote systemic health, or
(xi) clean teeth and oral cavity;
wherein the method comprises applying an effective amount of the oral care mouth rinse composition to the oral cavity of a subject in need thereof.

In another aspect, the present invention provides a method (Method 2) of stabilizing ionic tin in an oral care mouth rinse composition comprising at least one source of ionic tin, wherein the method comprises formulating the oral care mouth rinse composition of the invention. In preferred embodiments, the present invention provides Methods as follows:
2.1 Method 2, wherein the concentration of ionic tin in the oral care mouth rinse composition is from 0.01 to 0.10 weight % based on the total weight of the oral care mouth rinse composition.
2.2 Method 2 *et seq.* wherein the composition further comprises 0.10 to 0.25 weight % amine fluoride.
2.3 Method 2.2, wherein the amine fluoride is selected from N,N,N'-tris(2-hydroxyethyl)-N'-octadecyl-1,3-diaminopropane dihydrofluoride, 9-octadecenylamine hydrofluoride, or a combination thereof.
2.4 Method 2.2, wherein the amine fluoride is a mixture of N,N,N'-tris(2-hydroxyethyl)-N'-octadecyl-1,3-diaminopropane dihydrofluoride, and 9-octadecenylamine hydrofluoride.

In another aspect, the present invention provides a method (Method 3) of reducing staining of surfaces in the oral cavity resulting from use of an oral care mouth rinse comprising at least one source of ionic tin, wherein the method comprises formulating the oral care mouth rinse composition to comprise at least one aliphatic di- or tri-carboxylic acid in free or salt form as defined in claim 1 in an amount of from 0.05 to 0.30 weight % based on the total weight of the oral care composition.

In preferred embodiments, the present invention provides methods as follows:
3.1 Method 3, wherein the concentration of ionic tin in the oral care mouth rinse composition is from 0.01 to 0.10 weight % based on the total weight of the oral care mouth rinse composition.
3.2 Method 3 or 3.1, wherein the composition further comprises 0.10 to 0.25 weight % amine fluoride.
3.3 Method 3.2, wherein the amine fluoride is selected from N,N,N'-tris(2-hydroxyethyl)-N'-octadecyl-1,3-diaminopropane dihydrofluoride, 9-octadecenylamine hydrofluoride, or a combination thereof.
3.4 Method 3.2, wherein the amine fluoride is a mixture of N,N,N'-tris(2-hydroxyethyl)-N'-octadecyl-1,3-diaminopropane dihydrofluoride, and 9-octadecenylamine hydrofluoride.

### EXAMPLES

### Example 1

Mouth rinse formulations are prepared by mixing water, powdered ingredients and colorants in a vessel and stirring to dissolve. Stannous fluoride is dissolved in concentrated amine fluoride solution and added to the vessel. This mixture is stirred until homogeneous. Aroma, surfactants and all other remaining ingredients are added and the mixture stirred until the solution is clear. If necessary, the pH is adjusted.

Mouth rinse formulations A and B (Table 1) were prepared and evaluated in terms of their appearance and texture after ageing by storage at either 25 °C or 40 °C.

**Table 1**

| | **Formulation A*** | **Formulation B** |
|---|---|---|
| Malic acid | 0.00 | 0.20 |
| Xylitol | 0.85 | 0.85 |
| PVP (MW 58,000) | 0.30 | 0.30 |
| Emulsifier | 0.25 | 0.25 |
| Stannous fluoride | 0.05 | 0.05 |
| Amine fluoride | 0.17 | 0.17 |
| Water, sweetener, aroma, color, pH adjuster | QS | QS |

| | | |
|---|---|---|
| * not claimed | | |

The formulations were evaluated using a 4 point scale from 4 (best) to 1 (fail).

**Table2**

| **Formula** | **Appearance (color) after 3 months at 25 °C** | **Appearance (color) after 3 months at 40 °C** | **Appearance (color) after 6 months at 25 °C** | **Appearance (color) after 6 months at 40 °C** |
|---|---|---|---|---|
| A | Pass (3) | Pass (3) | Pass (3) | Pass (3) |
| | Light blue solution | Light blue solution | Light blue solution | Light blue solution with green shade |
| B | Pass (3) | Pass (3) | Pass (4) | Pass (4) |
| | Light blue solution | Light blue solution | | |

**Table 3**

| **Formula** | **Appearance (texture) after 3 months at 25 °C** | **Appearance (texture) after 3 months at 40 °C** | **Appearance (texture) after 6 months at 25 °C** | **Appearance (texture) after 6 months at 40 °C** |
|---|---|---|---|---|
| A | Pass (3) | Pass (2) | Pass(2) | Fail (1) |
| | Slightly turbid solution with traces of sediments | Slightly turbid solution with some sediments | Slightly turbid solution with slight sediments | Almost turbid solution with some sediments |
| B | Pass (3) | Pass (3) | Pass (4) | Pass (4) |
| | Borderline Clear solution without sediments | Borderline Clear solution without sediments | Clear solution without sediments | Clear solution without sediments |

These results demonstrate that addition of malic acid to a stannous containing mouth rinse reduces the formation of turbidity and reduce sediment formation. The malic acid containing formulations stay clear without sediment even after accelerated ageing at 40 °C for 6 months.

### Example 2

Additional mouthrinse formulations C through H are similarly compared as to color and texture during accelerated aging at 40 °C for 6 months. The results are shown in Table 3. Each formulation C through H has the same composition as formulation B, above, except for the malic acid content. It is found that all six of these additional formulations remain light blue solutions throughout the 6-months, however, it is found that a malic acid content of from 0.3 to 0.5% results in a clear solution at 6-months, but 0.05-0.1% malic acid does not.

**Table 4**

| **Formula** | **Appearance (texture) after 3 months at 40 °C** | **Appearance (texture) after 6 months at 40 °C** |
|---|---|---|
| C | Pass (2) | Fail (1) |
| (0.01% Malic Acid)* | Slightly turbid solution | Turbid solution |
| D | Pass (2) | Fail (1) |
| (0.05% Malic Acid)* | Slightly turbid solution | Turbid solution |
| E | Pass (4) | Pass (3) |
| (0.10% Malic Acid)* | Clear solution | Minimally turbid solution |
| F | Pass (4) | Pass (4) |
| (0.30% Malic Acid)* | Clear solution | Clear solution |
| G | Pass (4) | Pass (4) |
| (0.40% Malic Acid)* | Clear solution | Clear solution |
| H | Pass (4) | Pass (4) |
| (0.50% Malic Acid)* | Clear solution | Clear solution |

| | | |
|---|---|---|
| * not claimed | | |

## Claims

1. An oral care mouth rinse composition comprising
(i) at least one source of ionic tin;
(ii) at least one aliphatic di- or tri-carboxylic acid in free or salt form wherein the at least one aliphatic di- or tri-carboxylic acid in free or salt form is present in an amount of from 0.05 to 0.30 weight % based on the total weight of the oral care composition, wherein the at least one aliphatic di- or tri-carboxylic acid comprises malic acid in an amount of 0.12 to 0.25 weight % based on the total weight of the oral care composition; and
(iii) amine fluoride.

2. The oral care mouth rinse composition of claim 1 comprising 0.12 to 0.15 weight % malic acid.

3. The oral care composition of any preceding claim wherein the source of ionic tin is selected from the group comprising stannous ion sources, stannic ion sources and combinations thereof.

4. The oral care mouth rinse composition of any preceding claim wherein the source of ionic tin is selected from the group comprising stannous fluoride, stannous chloride, stannic fluoride, stannic chloride, stannic acetate, stannous acetate and combinations thereof.

5. The oral care mouth rinse composition of any preceding claim wherein the composition comprises at least one stannous ion source.

6. The oral care mouth rinse composition of any preceding claim wherein the source of ionic tin is selected from the group comprising stannous fluoride, stannous chloride, stannous acetate and combinations thereof.

7. The oral care mouth rinse composition of any preceding claim wherein the concentration of ionic tin is from 0.01 to 0.10 weight % based on the total weight of the oral care mouth rinse composition, optionally wherein the concentration of ionic tin is from 0.02 to 0.08 weight % based on the total weight of the oral care mouth rinse composition, further optionally wherein the concentration of ionic tin is from 0.03 to 0.06 weight % based on the total weight of the oral care mouth rinse composition, further optionally wherein the concentration of ionic tin is 0.04 weight % based on the total weight of the oral care mouth rinse composition.

8. The oral care mouth rinse composition of any preceding claim wherein the source of ionic tin comprises stannous fluoride.

9. The oral care mouth rinse composition of any preceding claim comprising amine fluoride in an amount corresponding to 45 to 1500 ppm fluoride based on the total weight of the composition.

10. The oral care mouth rinse composition of any preceding claim comprising 0.06 to 0.33 weight % amine fluoride based on the total weight of the composition.

11. The oral care mouth rinse composition of any preceding claim comprising 0.10 to 0.25 weight % malic acid, 0.04 to 0.08 weight % stannous fluoride and 0.10 to 0.25 weight % N,N,N'-tris(2-hydroxyethyl)-N'-octadecyl-1,3-diaminopropane dihydrofluoride.

12. An oral care mouth rinse composition according to any of claims 1 to 11 for use in a method to
(i) reduce levels of acid producing bacteria,
(ii) reduce or inhibit microbial biofilm formation in the oral cavity,
(iii) reduce or inhibit plaque formation in the oral cavity,
(iv) promote systemic health, or
(v) clean teeth and oral cavity,
comprising applying an effective amount of said oral care mouth rinse composition to the oral cavity of a subject in need thereof.

13. An oral care mouth rinse composition according to any of claims 1 to 11 for use in a method to
(i) reduce or inhibit formation of dental caries,
(ii) reduce, repair or inhibit pre-carious lesions of the enamel,
(iii) reduce or inhibit demineralization and promote remineralization of the teeth,
(iv) reduce hypersensitivity of the teeth,
(v) reduce or inhibit gingivitis,
(vi) promote healing of sores or cuts in the oral cavity,
(vii) reduce levels of acid producing bacteria,
(viii) reduce or inhibit microbial biofilm formation in the oral cavity,
(viii) reduce or inhibit plaque formation in the oral cavity, or
(ix) promote systemic health.

14. A method of stabilizing ionic tin in an oral care mouth rinse composition comprising at least one source of ionic tin, wherein the method comprises formulating the oral care mouth rinse composition to comprise at least one aliphatic di- or tri-carboxylic acid in free or salt form as defined in claim 1 in an amount as defined in claim 1.

15. A method of reducing staining of surfaces in the oral cavity resulting from use of an oral care mouth rinse comprising at least one source of ionic tin, wherein the method comprises formulating the oral care mouth rinse composition to comprise at least one aliphatic di- or tricarboxylic acid in free or salt form as defined in claim 1 in an amount as defined in claim 1.

## Patentansprüche

1. Mundspülungszusammensetzung zur Mundpflege, umfassend
(i) mindestens eine Quelle ionischen Zinns;
(ii) mindestens eine aliphatische Di- oder Tricarbonsäure in freier Form oder in Salzform, wobei die mindestens eine aliphatische Di- oder Tricarbonsäure in freier Form oder Salzform in einer Menge von 0,05 bis 0,30 Gew.-% bezogen auf das Gesamtgewicht der Mundpflegezusammensetzung vorliegt, wobei die mindestens eine aliphatische Di- oder Tricarbonsäure Äpfelsäure in einer Menge von 0,12 bis 0,25 Gew.-% bezogen auf das Gesamtgewicht der Mundpflegezusammensetzung umfasst; und
(iii) Aminfluorid.

2. Mundspülungszusammensetzung zur Mundpflege nach Anspruch 1, die 0,12 bis 0,15 Gew.-% Äpfelsäure umfasst.

3. Mundpflegezusammensetzung nach einem der vorhergehenden Ansprüche, wobei die Quelle ionischen Zinns aus der Gruppe umfassend Zinn(II)-Ionenquellen, Zinn(IV)-Ionenquellen und Kombinationen aus denselben ausgewählt ist.

4. Mundspülungszusammensetzung zur Mundpflege nach einem der vorhergehenden Ansprüche, wobei die Quelle ionischen Zinns aus der Gruppe umfassend Zinn(II)-fluorid, Zinn(II)-chlorid, Zinn(IV)-fluorid, Zinn(IV)-chlorid, Zinn(IV)-acetat, Zinn(II)-acetat und Kombinationen aus denselben ausgewählt ist.

5. Mundspülungszusammensetzung zur Mundpflege nach einem der vorhergehenden Ansprüche, wobei die Zusammensetzung mindestes eine Zinn(II)-Ionenquelle umfasst.

6. Mundspülungszusammensetzung zur Mundpflege nach einem der vorhergehenden Ansprüche, wobei die Quelle ionischen Zinns aus der Gruppe umfassend Zinn(II)-fluorid, Zinn(II)-chlorid, Zinn(II)-acetat und Kombinationen aus denselben ausgewählt ist.

7. Mundspülungszusammensetzung zur Mundpflege nach einem der vorhergehenden Ansprüche, wobei die Konzentration ionischen Zinns zwischen 0,01 bis 0,10 Gew.-% bezogen auf das Gesamtgewicht der Mundspülungszusammensetzung zur Mundpflege liegt, optional, wobei die Konzentration ionischen Zinns zwischen 0,02 bis 0,08 Gew.-% bezogen auf das Gesamtgewicht der Mundspülungszusammensetzung zur Mundpflege liegt, ferner optional, wobei die Konzentration ionischen Zinns zwischen 0,03 bis 0,06 Gew.-% bezogen auf das Gesamtgewicht der Mundspülungszusammensetzung zur Mundpflege liegt, ferner optional, wobei die Konzentration ionischen Zinns zwischen 0,04 Gew.-% bezogen auf das Gesamtgewicht der Mundspülungszusammensetzung zur Mundpflege beträgt.

8. Mundspülungszusammensetzung zur Mundpflege nach einem der vorhergehenden Ansprüche, wobei die Quelle ionischen Zinns Zinn(II)-fluorid umfasst.

9. Mundspülungszusammensetzung zur Mundpflege nach einem der vorhergehenden Ansprüche, die Aminfluorid in einer Menge umfasst, die 45 bis 1500 ppm Fluorid bezogen auf das Gesamtgewicht der Zusammensetzung entspricht.

10. Mundspülungszusammensetzung zur Mundpflege nach einem der vorhergehenden Ansprüche, die 0,06 bis 0,33 Gew.-% Aminfluorid bezogen auf das Gesamtgewicht der Zusammensetzung umfasst.

11. Mundspülungszusammensetzung zur Mundpflege nach einem der vorhergehenden Ansprüche, die 0,10 bis 0,25 Gew.-% Äpfelsäure, 0,04 bis 0,08 Gew.-% Zinn(II)-fluorid und 0,10 bis 0,25 Gew.-% N,N,N'-Tris(2-hydroxyethyl)-N'-octadecyl-1,3-diaminopropandihydrofluorid umfasst.

12. Mundspülungszusammensetzung zur Mundpflege nach einem der Ansprüche 1 bis 11 zur Verwendung in einem Verfahren zum
(i) Reduzieren der Konzentration säureproduzierender Bakterien,
(ii) Reduzieren oder Hemmen der Bildung von mikrobiellem Biofilm in der Mundhöhle,
(iii) Reduzieren oder Hemmen von Plaquebildung in der Mundhöhle,
(iv) Fördern systemischer Gesundheit, oder
(v) Reinigen der Zähne und Mundhöhle,
umfassend das Anwenden einer wirksamen Menge der Mundspülungszusammensetzung zur Mundpflege auf die Mundhöhle eines Subjekts, die diese benötigt.

13. Mundspülungszusammensetzung zur Mundpflege nach einem der Ansprüche 1 bis 11 zur Verwendung in einem Verfahren zum
(i) Reduziere oder Hemmen der Bildung von Zahnkaries,
(ii) Reduzieren, Reparieren oder Hemmen von vorkariösen Läsionen des Zahnschmelzes,
(iii) Reduzieren oder Hemmen von Demineralisierung und Fördern von Remineralisierung der Zähne,
(iv) Reduzieren von Überempfindlichkeit der Zähne,
(v) Reduzieren oder Hemmen von Gingivitis,
(vi) Fördern der Heilung von Wunden und Schnitten in der Mundhöhle,
(vii) Reduzieren der Konzentration säureproduzierender Bakterien,
(viii) Reduzieren oder Hemmen der Bildung von mikrobiellem Biofilm in der Mundhöhle,
(viii) Reduzieren oder Hemmen von Plaquebildung in der Mundhöhle, oder
(ix) Fördern systemischer Gesundheit.

14. Verfahren zum Stabilisieren von ionischem Zinn in einer Mundspülungszusammensetzung zur Mundpflege, die mindestens eine Quelle ionischen Zinns umfasst, wobei das Verfahren das Formulieren der Mundspülungszusammensetzung zur Mundpflege umfasst, um mindestens eine aliphatische Di- oder Tricarbonsäure in freier Form oder in Salzform nach Anspruch 1 in einer Menge nach Anspruch 1 zu umfassen.

15. Verfahren zum Reduzieren der Verfärbung von Oberflächen in der Mundhöhle, die aus der Verwendung einer Mundpflegespülung resultierte, die mindestens eine Quelle ionischen Zinns umfasst, wobei das Verfahren das Formulieren der Mundspülungszusammensetzung zur Mundpflege umfasst, um mindestens eine aliphatische Di- oder Tricarbonsäure in freier Form oder in Salzform nach Anspruch 1 in einer Menge nach Anspruch 1 zu umfassen.

## Revendications

1. Composition d'eau dentaire pour soins buccaux comprenant
(i) au moins une source d'étain ionique ;
(ii) au moins un acide di- ou tri-carboxylique aliphatique sous forme libre ou sous forme de sel, l'au moins un acide di- ou tri-carboxylique aliphatique sous forme libre ou sous forme de sel étant présent en une quantité de 0,05 à 0,30 % en poids sur la base du poids total de la composition pour soins buccaux, l'au moins un acide di- ou tri-carboxylique aliphatique comprenant de l'acide malique en une quantité de 0,12 à 0,25 % en poids sur la base du poids total de la composition pour soins buccaux ; et
(iii) du fluorure d'amine.

2. Composition d'eau dentaire pour soins buccaux selon la revendication 1, comprenant 0,12 à 0,15 % en poids d'acide malique.

3. Composition pour soins buccaux selon l'une quelconque des revendications précédentes, dans laquelle la source d'étain ionique est choisie dans le groupe comprenant les sources d'ions stanneux, les sources d'ions stanniques et des combinaisons de celles-ci.

4. Composition d'eau dentaire pour soins buccaux selon l'une quelconque des revendications précédentes, dans laquelle la source d'étain ionique est choisie dans le groupe comprenant le fluorure stanneux, le chlorure stanneux, le fluorure stannique, le chlorure stannique, l'acétate stannique, l'acétate stanneux et des combinaisons de ceux-ci.

5. Composition d'eau dentaire pour soins buccaux selon l'une quelconque des revendications précédentes, dans laquelle la composition comprend au moins une source d'ions stanneux.

6. Composition d'eau dentaire pour soins buccaux selon l'une quelconque des revendications précédentes, dans laquelle la source d'étain ionique est choisie dans le groupe comprenant le fluorure stanneux, le chlorure stanneux, l'acétate stanneux et des combinaisons de ceux-ci.

7. Composition d'eau dentaire pour soins buccaux selon l'une quelconque des revendications précédentes, dans laquelle la concentration d'étain ionique est de 0,01 à 0,10 % en poids sur la base du poids total de la composition d'eau dentaire pour soins buccaux, éventuellement dans laquelle la concentration d'étain ionique est de 0,02 à 0,08 % en poids sur la base du poids total de la composition d'eau dentaire pour soins buccaux, éventuellement en outre dans laquelle la concentration d'étain ionique est de 0,03 à 0,06 % en poids sur la base du poids total de la composition d'eau dentaire pour soins buccaux, éventuellement en outre dans laquelle la concentration d'étain ionique est de 0,04 % en poids sur la base du poids total de la composition d'eau dentaire pour soins buccaux.

8. Composition d'eau dentaire pour soins buccaux selon l'une quelconque des revendications précédentes, dans laquelle la source d'étain ionique comprend du fluorure stanneux.

9. Composition d'eau dentaire pour soins buccaux selon l'une quelconque des revendications précédentes, comprenant du fluorure d'amine en une quantité correspondant à 45 à 1500 ppm de fluorure sur la base du poids total de la composition.

10. Composition d'eau dentaire pour soins buccaux selon l'une quelconque des revendications précédentes, comprenant 0,06 à 0,33 % en poids de fluorure d'amine sur la base du poids total de la composition.

11. Composition d'eau dentaire pour soins buccaux selon l'une quelconque des revendications précédentes, comprenant 0,10 à 0,25 % en poids d'acide malique, 0,04 à 0,08 % en poids de fluorure stanneux et 0,10 à 0,25 % en poids de dihydrofluorure de N,N,N'-tris (2-hydroxyéthyl)-N'-octadécyle-1,3-diaminopropane.

12. Composition d'eau dentaire pour soins buccaux selon l'une quelconque des revendications 1 à 11, destinée à être utilisée dans un procédé pour
(i) réduire les niveaux de bactéries productrices d'acide,
(ii) réduire ou inhiber la formation de biofilm microbien dans la cavité buccale,
(iii) réduire ou inhiber la formation de plaque dans la cavité buccale,
(iv) promouvoir la santé systémique, ou
(v) nettoyer les dents et la cavité buccale,
comprenant l'application d'une quantité efficace de ladite composition d'eau dentaire pour soins buccaux dans la cavité buccale d'un sujet en ayant besoin.

13. Composition d'eau dentaire pour soins buccaux selon l'une quelconque des revendications 1 à 11, destinée à être utilisée dans un procédé pour
(i) réduire ou inhiber la formation de caries dentaires,
(ii) réduire, réparer ou inhiber les lésions précarieuses de l'émail,
(iii) réduire ou inhiber la déminéralisation et favoriser la reminéralisation des dents,
(iv) réduire l'hypersensibilité des dents,
(v) réduire ou inhiber la gingivite,
(vi) favoriser la cicatrisation des plaies ou coupures de la cavité buccale,
(vii) réduire les niveaux de bactéries productrices d'acide,
(viii) réduire ou inhiber la formation de biofilm microbien dans la cavité buccale
(ix) réduire ou inhiber la formation de plaque dans la cavité buccale, ou
(x) favoriser la santé systémique.

14. Procédé de stabilisation de l'étain ionique dans une composition d'eau dentaire pour soins buccaux comprenant au moins une source d'étain ionique, le procédé comprenant la formulation de la composition d'eau dentaire pour soins buccaux de manière à comprendre au moins un acide di- ou tri-carboxylique aliphatique sous forme libre ou sous forme de sel tel que défini dans la revendication 1 en une quantité telle que définie dans la revendication 1.

15. Procédé de réduction de la coloration des surfaces dans la cavité buccale résultant de l'utilisation d'une eau dentaire pour soins buccaux comprenant au moins une source d'étain ionique, le procédé comprenant la formulation de la composition d'eau dentaire pour soins buccaux de manière à comprendre au moins un acide di- ou tricarboxylique aliphatique sous forme libre ou sous forme de sel tel que défini dans la revendication 1 en une quantité telle que définie dans la revendication 1.
